# EUROPEAN PATENT SPECIFICATION

(11) **EP 3 856 122 B1**
(45) Date of publication and mention of the grant of the patent: **26.03.2025**
(21) Application number: 19867961.5
(22) Date of filing: 22.08.2019
(51) Int. Cl.: A61J 15/00, A61M 25/02, A61M 25/16, A61M 25/18, A61M 31/00, A61M 39/00

(54) **STOMA SITE PROTECTION DEVICES**
VORRICHTUNG ZUM SCHUTZ EINER OSTOMIESTELLE
DISPOSITIFS DE PROTECTION DE SITE DE STOMIE

(30) Priority: 26.09.2018 US 201862736637 P
(43) Date of publication of application: 04.08.2021
(73) Proprietor: The Cleveland Clinic Foundation, Cleveland, OH 44195 (US)
(72) Inventor: BLUMROSEN, Eric Yudelevich, Cleveland, Ohio 44195 (US); WILLIAMS, Andrew G., Cleveland, Ohio 44195 (US); GAO, Shengqiang, Cleveland, Ohio 44195 (US)
(74) Representative: Forresters IP LLP
(86) International application number: PCT/US2019/047658
(87) International publication number: WO 2020/068321

(56) References cited:
- WO-A1-96/36378
- US-A- 4 668 227
- US-A- 5 318 543
- US-A1- 2006 052 752
- US-A1- 2013 165 862
- US-A1- 2013 165 862
- US-A1- 2017 050 004

## Description

### TECHNICAL FIELD

The present disclosure relates to stoma site protection devices.

### BACKGROUND

Feeding tubes such as a jejunostomy tube (J-tube) and a gastrostomy tube (G-tube) may be used to provide nutritional support for users who are on prolonged artificial ventilation, have suffered central nervous system trauma or other condition in which the user is unable to maintain weight without assistance. The tubes are typically secured by an external retention ring on the outside skin surface and an internal retention feature, such as a small inflated balloon or bumper inside the stomach (G-tube) or small intestine (J-tube). The feeding tube may remain in place for several months either in a hospital or home setting.

External retention rings may slip, which can break the seal at the stomach /intestinal wall and allows acidic gastric fluid to leak onto the skin. This leakage can cause skin irritation and wounds that can progress to infection, requiring medical intervention or additional operations. Leakage may also occur around drainage tubes, such as chest tubes and surgical drains. These types of tubes generally remain in place for several days while the user is in the hospital. As such, a need exists for a device that addresses the problem of gastric fluid leakage as well as leakage of other fluids from tubes that are inserted in a patient.

US 2013/165862 discloses a feeding device having an improved base adapted to be deployed outside the human body and a tube which is adapted to be deployed within a lumen or cavity of the body by insertion through a stoma from outside the body is disclosed.

### BRIEF DESCRIPTION OF THE DRAWINGS

FIG. 1 is a perspective view of a stoma site protection device according to an aspect of the present disclosure.
FIG. 2 is a cross-sectional view of the stoma site protection device of FIG. 1.
FIG. 3 is a perspective view of a stoma site protection device according to an aspect of the present disclosure.
FIG. 4 is a perspective view of a stoma site protection device and stoma tract patency device according to an aspect of the present disclosure.
FIG. 5 is a perspective view of a stoma site protection device according to an aspect of the present disclosure.
FIG. 6 is a perspective view of a stoma site protection device according to an aspect of the present disclosure.
FIG. 7 is a perspective view of a stoma site protection device in an unexpanded state according to an aspect of the present disclosure.
FIG. 8 is a perspective view of the stoma site protection device of FIG. 7 in an expanded state.
FIG. 9 is a perspective view of an external bumper of an assembly or kit according to an aspect of the present disclosure.
FIG. 10 is a perspective view of a bandage of an assembly or kit according to an aspect of the present disclosure.
FIG. 11 is a perspective view of a bandage of an assembly or kit according to an aspect of the present disclosure.
FIG. 12 is a perspective view of a bandage of an assembly or kit according to an aspect of the present disclosure.
FIG. 13 is a perspective view of a feeding tube assembly according to an aspect of the present disclosure.
FIG. 14 is a cross-sectional view of the feeding tube assembly of FIG. 13.
FIG. 15 is a cross-sectional view of an integrated medical device including a bandage, an external bumper and a stoma site protection device according to an aspect of the present disclosure.
FIG. 16 is a perspective view of an inner portion of a stoma site protection device according to an aspect of the present disclosure.
FIG. 17 is a cross-sectional view of a feeding tube assembly according to an aspect of the present disclosure.
FIG. 18 is an exploded view of a stoma site protection device and accompanying components and depicts an image of a stoma site protection device and accompanying components during a step of assembly according to an aspect of the present disclosure.
FIG. 19 depicts an image of a stoma site protection device and accompanying components during a step of assembly according to an aspect of the present disclosure.
FIG. 20 depicts an image of a stoma site protection device and accompanying components during a step of assembly according to an aspect of the present disclosure.
FIG. 21 depicts an image of a stoma site protection device and accompanying components during a step of assembly according to an aspect of the present disclosure.
FIG. 22 is a side view of a prior art feeding tube inserted in a user's stoma, stoma tract and stomach.
FIG. 23 depicts an image of a stoma site protection device and other assembled components during a step of insertion into a stoma, stoma tract, and stomach according to an aspect of the present disclosure.
FIG. 24 depicts an image of a stoma site protection device and other assembled components during a step of insertion into a stoma, stoma tract, and stomach according to an aspect of the present disclosure.
FIG. 25 depicts an image of a stoma site protection device and other assembled components during a step of insertion into a stoma, stoma tract, and stomach according to an aspect of the present disclosure.
FIG. 26 depicts an image of a stoma site protection device and other assembled components in their final position on a skin surface and in a stoma, stoma tract, and stomach according to an aspect of the present disclosure.
FIG. 27 is a cross-sectional view of the stoma site protection device and assembled components of FIG. 26.

### DETAILED DESCRIPTION

The present disclosure refers to the terms "upper," "lower," "top," and "bottom" with respect to certain components. These terms refer to configuration of the components as illustrated in the drawings and as indicated by the character references. Further, as used herein with respect to a described element, the terms "a," "an," and "the" include at least one or more of the described element unless otherwise indicated. Further, the term "or" refers to "and/or" and "combinations thereof" unless otherwise indicated. In addition, when an element is referred to as being "over," "on," "attached" to, "connected" to, "coupled" to etc., another element, it can be directly over, on, attached to, connected to, coupled to, etc. the other element or intervening elements may also be present. In contrast, when an element is referred to as being, for example, "directly over," "directly on," "directly attached" to, "directly connected" to or "directly coupled" to another element, there are no intervening elements present. By "integral" or "integrated" is meant that the described components are fabricated as one piece or multiple pieces affixed during manufacturing or the described components are otherwise not separable using a normal amount of force without damaging the integrity (i.e. tearing) of the described components. A normal amount of force is the amount of force a user would use to remove a component meant to be separated from another component without damaging the components.

The present disclosure relates to stoma site protection devices and kits to prevent leakages of fluid from a stoma. Disclosed devices and kits include components and/or features that create a barrier to leakage at a stoma site. A stoma is an artificial opening made in a patient's skin to create a tract from an area inside the body to the patient's outer skin surface. A stoma is not a surgical incision site that is intended to be permanently closed after completion of the surgical procedure but rather an opening that is intended to remain open for a period of time after the stoma is created so that a percutaneous tube can be inserted through the stoma and remain in the patient for a period of time to deliver, remove, process, or otherwise accept fluid from the patient's body. A stoma can lead to a hollow organ or cavity such as organs of the gastrointestinal tract or the pleural cavity of the lungs. The below disclosure is described with respect to a feeding tube, which is a vessel through which fluids are delivered as well as through which fluids are drained from internal structures such as the stomach or the intestines via a stoma in the patient's body. However, devices and kits as described herein apply to other types of tubes that are inserted into an artificial opening made in a patient's body that extend from an exterior surface of the patient's body to an internal structure of the patient's body as well as other percutaneous tubes. All stoma site protection devices and accompanying components as described herein are used for medical purposes and are therefore sterile. As used herein, a "patient" or "user" is a mammal, such as a human being.

As stated above, leakage of bodily fluid, such as bile or gastric acid, can occur at the stoma site irritating skin around the stoma site. Such stoma site leakage currently has been described as being due to an incorrectly sized tube, a low water level in the internal retention element (such as a balloon), the stomach being too full of gastric acid or formula/food, and/or stomach gas. However, without wishing to be bound by theory, it is believed that a significant cause of leakage around the stoma site is, in actuality, the friction from the tube rubbing against the patient's skin at the stoma site during regular activity causing irritation and eventually creating a fissure between the skin and the end organ or cavity from which bile or gastric acid can leak. Basic bandages are not enough to keep tubes from moving which results in irritation and increased stoma size, resulting in leakage around the feeding tube. Daily activity, weight loss or gain, users tugging at insecure tubes, tube movement with contractions of the digestive system, for example, can cause tubes to move resulting in leakage. Such movement can break the seal at the stomach/intestinal wall and expand the diameter of the stoma tract through which the tube extends resulting in gastric/biliary fluid leakage onto the skin. This leakage can cause skin irritation and wounds that can progress to infection, cellulitis or abscesses. Such conditions can require medical intervention such emergency room visits, hospital admission, antibiotic use, or additional operations/tube exchange. Leakage may also occur around drainage tubes, such as chest tubes and surgical drains. Stoma site protection devices as disclosed herein can address this cause by allowing the tube to move freely without rubbing against the patient's skin. Such stoma site protection devices effectively act as bearings reducing the friction between the tube and the patient's skin.

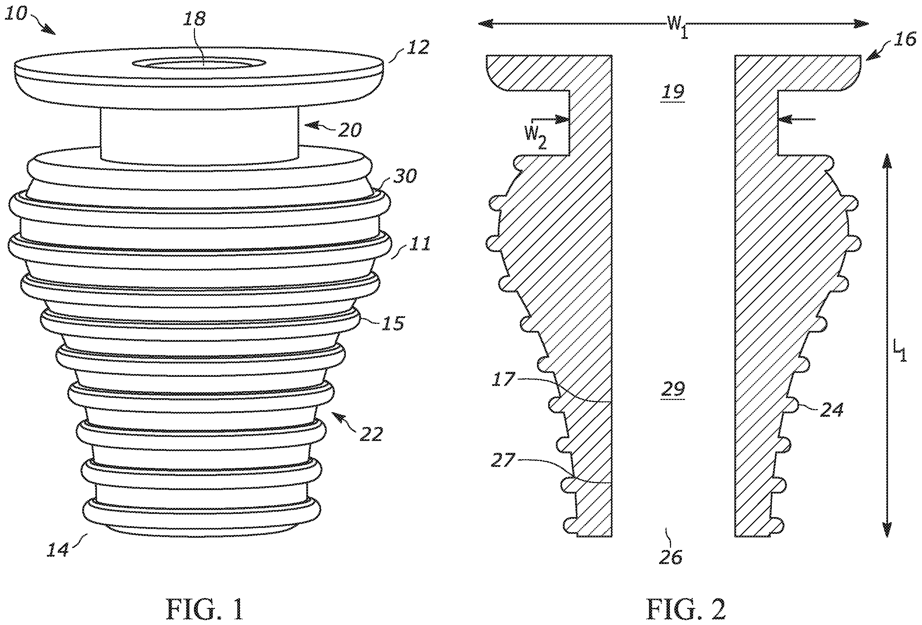

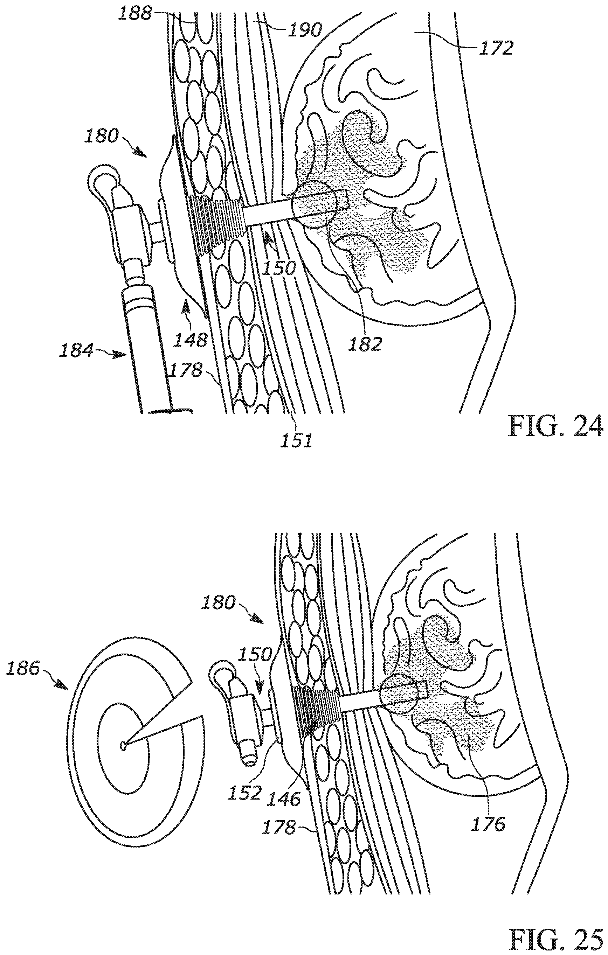

Referring to FIGs. 1 and 2, a stoma site protection device **10** comprises a body **11** having a proximal end **12,** a distal end **14,** an outer surface **15,** and an inner bearing surface **17.** Body **11** comprises a proximal collar **16** having a collar width **W₁** and defining a top opening **18**. An indented portion **20** extends distally from proximal collar **16** and has an indented portion width **W₂** that is less than collar width **W₁**. **W₁** of indented portion **20** can have a width that is substantially equal to the width of a stoma such that indented portion snugly conforms to and fits in stoma. The indented portion can be tubular in shape. As illustrated in FIG. 2, a lumen **19** can extend from top opening **18** to indented portion **20**. Body **11** also include a distal silicone plug **22** extending distally from indented portion **20**. Plug **22** can have a length **L₁** that allows plug **22** to be positioned within the portion of the stoma tract that is the subcutaneous fat layer above the patient's muscle or fascia layer and below the patient's outer skin surface as depicted in FIGs. 24-27. Plug **22** has an outer surface **24** and an inner bearing surface **27**. Inner bearing surface **27** can define a plug lumen **29** and can define a bottom opening **26**. The inner bearing surface can define a plug lumen having a diameter of between about 6 millimeters and about 10 millimeters in order to accommodate a percutaneous tube. Plug lumen **29** and bottom opening **26** are axially aligned with and in fluid communication with lumen **19** and top opening **18** of proximal collar **16** to form a single fluid lumen. As shown in FIGs. 1 and 2, outer surface **24** of plug **22** can include ridges **30** about a circumference thereof. The ridges can extend partially or fully about the circumference of the outer wall of the plug. Such ridges can facilitate purchase between the outer wall of the plug and the stoma tract to provide a snug or tight fit of the plug in the stoma tract and prevent the plug from migrating or dislodging from the stoma tract.

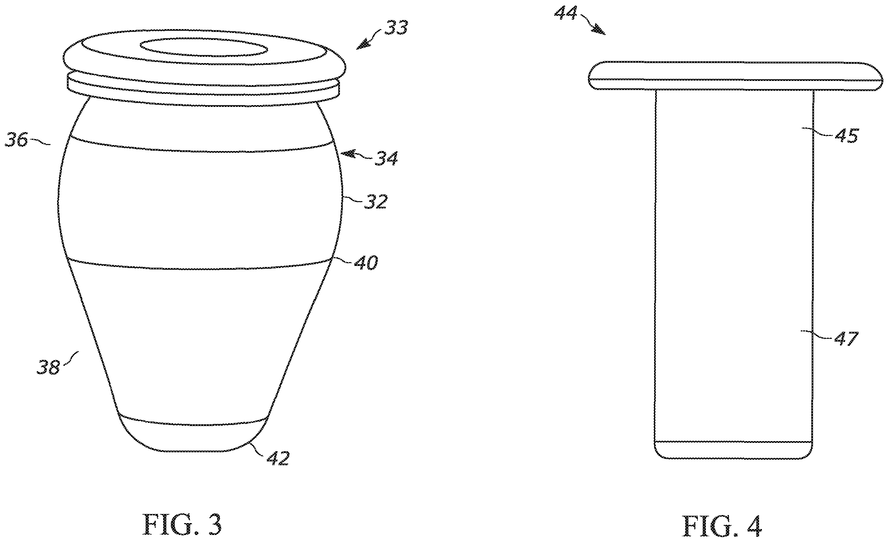

In other embodiments, as illustrated in FIG. 3, the outer surface **32** of a plug **34** of a stoma site protection device **33** can be smooth so as to minimize skin irritation in patients with sensitive skin, for example. Whether smooth or ridged, the plug can have a tapered or non-tapered shape. For example, referring to FIG. 3, the plug can have an upper portion **36** and a lower portion **38** with upper portion **36** having a bulbous shape and/or lower portion **38** tapering from a top end **40** to a bottom end **42** of lower portion **38**. The bulbous shape of upper portion **36** can increase the surface area of plug **34** that contacts the proximal surface of the stoma track to further secure the plug in the stoma tract and minimize leakage from the stoma as well. The tapered lower portion can allow for a stoma tract having a smaller diameter, easier insertion of the plug, and easier longitudinal adjustment of the plug if necessary.

FIG. 4 illustrates a stoma site protection device **44** where indented portion **45** and plug **47** have substantially the same uniform diameter along the length of the indented portion and the plug. In this configuration, plug **47** has a substantially non-tapered shape (*e.g*. the diameter of the plug is substantially the same along the length of the plug). Such a configuration can be used while a stoma site is developing, for example. Once a feeding tube is placed, the stoma tract starts maturing in about one to about two weeks and is usually well formed in about four to about six weeks. Such a taperless configuration mimics the path of a maturing stoma. Once the stoma is matured and fully developed, a patient or caregiver can place a stoma site protection device having a tapered plug, for example, into the stoma for greater stability. Such a configuration can benefit temporary as well as chronic feeding tube patients.

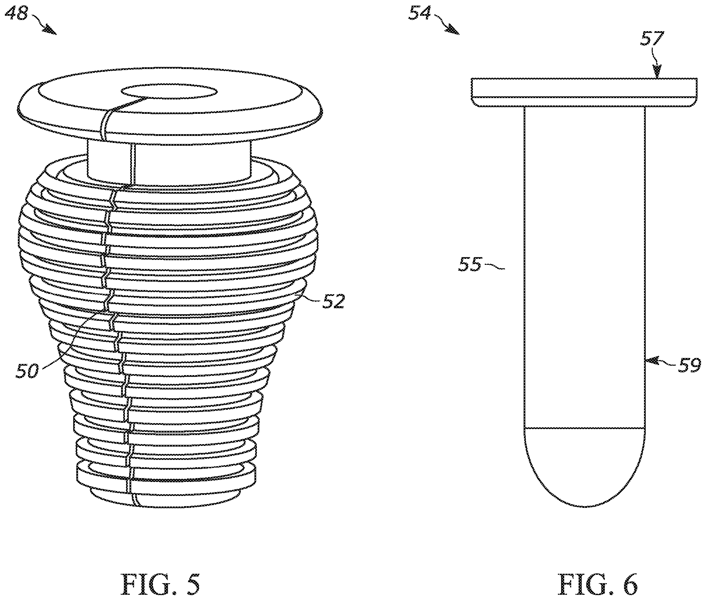

FIG. 5 illustrates a stoma site protection device **48** with a longitudinal slit **50** extending fully or partially along the outer wall **52** of the device. Such a slit allows the stoma site protection device to be placed around an existing percutaneous tube so that the tube does not need to be removed from the stoma. The device can be opened manually by a patient or caregiver, wrapped around the tube, and then closed. The end user can then slide the device distally along the tube into the stoma. Such a configuration can be useful for several different types of tubes, such as, for example, Dangler feeding tubes commonly used when developed a stoma site and other percutaneous tubes such as those used with ventricular assist devices (*e.g*. a left ventricular assist device (LVAD)).

FIG. 6 illustrates a non-cannulated stoma and stoma tract patency device **54** comprising a solid body **55** comprising a proximal collar **57** and a plug **59** extending distally from proximal collar **57.** Stoma and stoma tract patency device **54** is solid with no lumen extending therethrough. Such a configuration can be used to maintain a stoma site when a patient does not have an internal retention element, such as a balloon, inserted in the stomach or other organ or cavity for some period of time. Because the device is solid in its entirety, it provides enough rigidity to prevent the site from closing but also reduces skin irritation.

The inner bearing surface of a plug of a stoma site protection device has a Shore durometer hardness greater than the Shore durometer hardness of the outer wall so that the inner bearing wall is more rigid and the outer surface is more conformal. Such a configuration can allow for the feeding tube to have sufficient structural support within the plug such that the feeding tube stays in place but allows the plug to conform to the user's stoma tract, creating a stronger seal. In other words, the plug can be pliable enough to allow a feeding tube to move slightly as needed when the patient moves but still prevent leaks from occurring. The outer surface of a plug of a stoma site protection device is fabricated from a silicone material, so that the plug conforms to the stoma tract, adapts to varying stoma sizes, and achieves the appropriate fit and anchoring in the stoma tract. The stoma site protection device can be an integral, one-piece device.

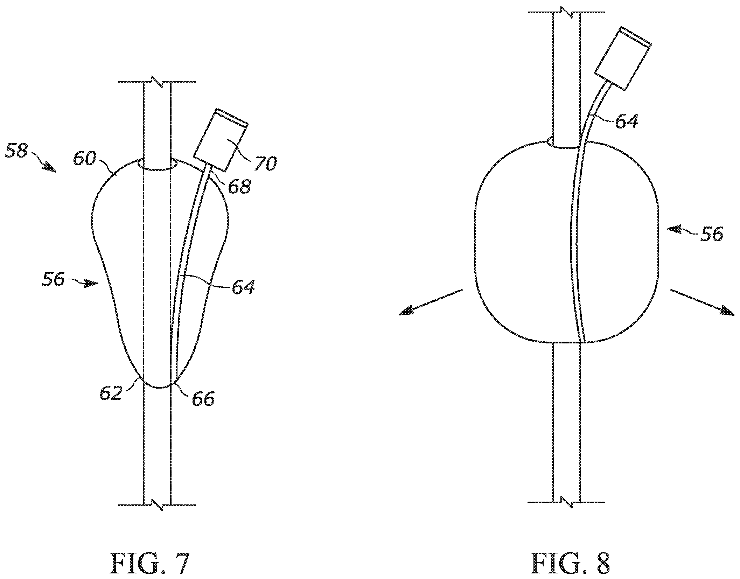

Referring to FIG. 7 and 8, a plug **56** of a stoma site protection device **58** can be a balloon having a top portion **60** and a bottom portion **62** and that can transition from an un-inflated state to an inflated state. Plug **56** can have an inflation lumen **64** with one end **66** in fluid communication with bottom portion **62** of plug **56** and another end **68** in fluid communication with an infusion port **70** at top portion **60** of plug **56**. Such a configuration allows the plug to be inflated upwards from the lowest point of the plug, thereby creating a seal as close as possible to the initial site of potential leakage in the digestive tract (such as the stomach or intestine), for example.

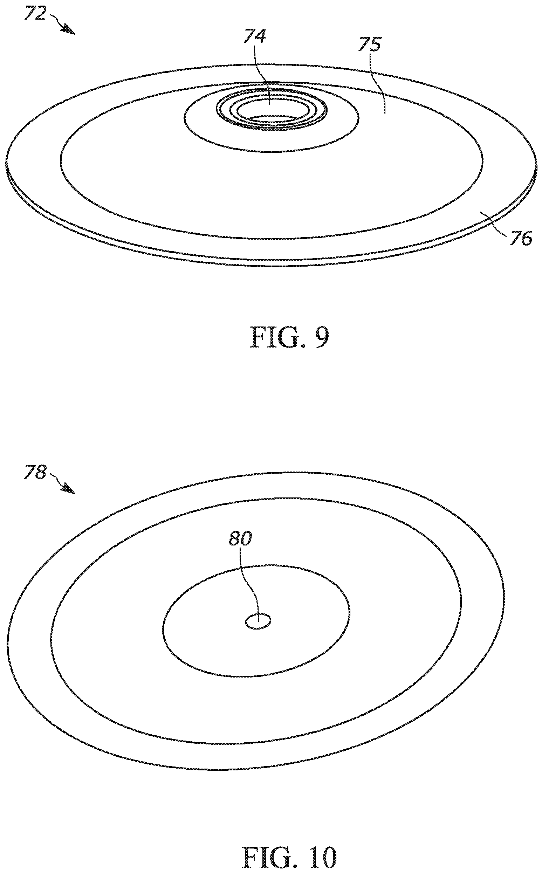

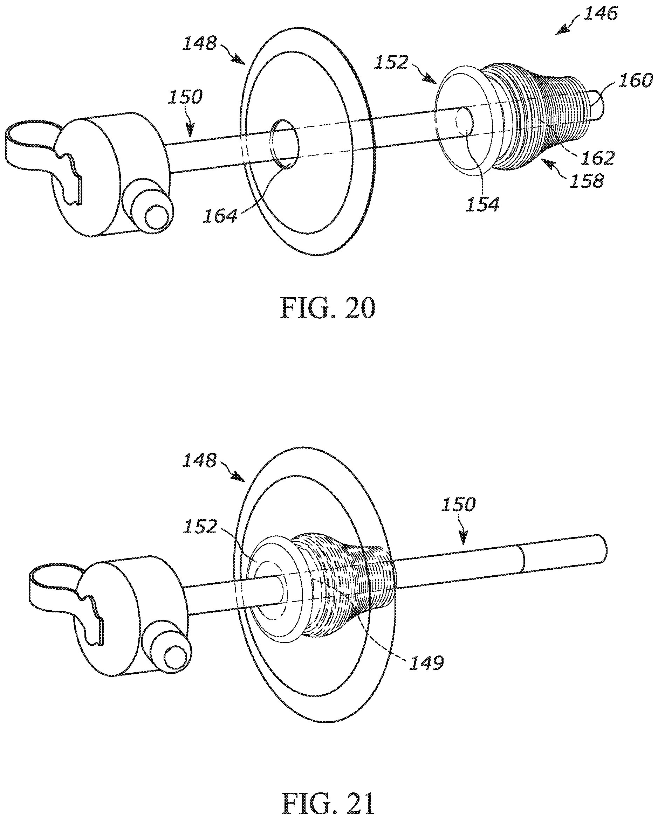

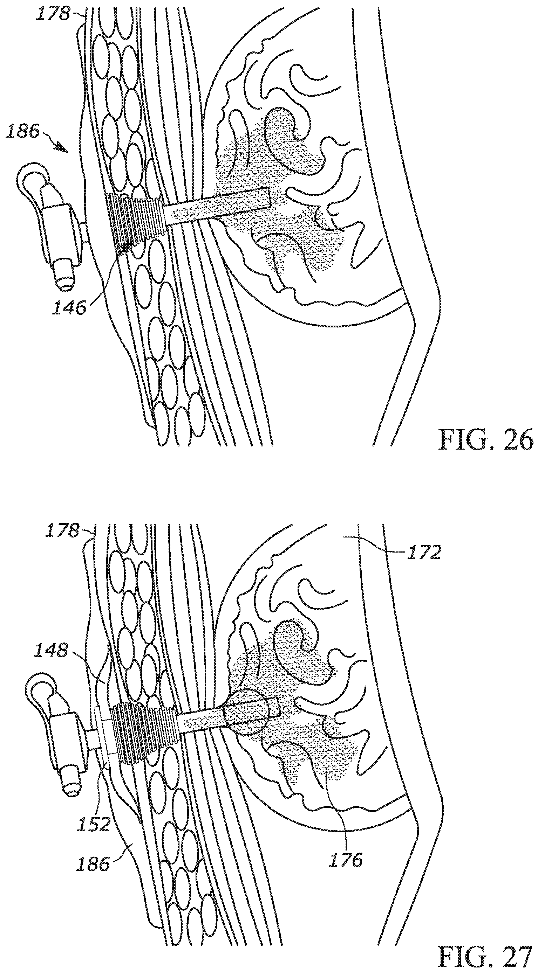

In certain aspects, a stoma site protection kit or assembly is provided that includes a stoma site protection device and other components that can improve the functionality of a feeding tube assembly or other medical tube assembly, such as by creating multiple seals of the stoma. For example, referring to FIG. 9, a stoma site protection assembly or kit can include a flexible bumper **72** that defines an opening **74** that can be axially aligned with the longitudinally extending lumen of a stoma site protection device in use and that is sized and configured to be placed about the indented portion of the body of a stoma site protection device as shown in FIGs. 21 and 27 and discussed in more detail below. As shown in FIG. 9, opening **74** can be surrounded by a dome shaped portion **75,** which can be surrounded by a flange **76.** In use, the bumper sits externally on the patient's skin preventing the stoma site protection device from slipping into the stoma and stabilizing the entire feeding tube assembly. For example, after the feeding tube is inserted in the patient, the bumper can be compressed over the stoma putting the entire tube assembly under tension preventing undesirable movement of the tube assembly. Dome shaped portion **75** ensures skin contact with the bumper is minimal and away from the stoma site (see *e.g*. FIG. 27) to prevent irritation at the stoma site. Skin contact can be limited to the area of the skin in contact with flange **76,** which is spaced from the stoma site in use (see *e.g*. FIG. 27). The bumper can have an outer diameter of between about 59 millimeters and about 63 millimeters to adequately cover the stoma site as well as stabilize the percutaneous tube. The bumper can have a height of between about 5 millimeters and about 9 millimeters.

Referring to FIGs. 10-12, a stoma site protection kit or assembly can include a bandage **78** defining an opening **80** extending therethrough to accommodate the proximal end of a feeding tube. Bandage can secure a stoma site protection device and a bumper (in embodiments including a bumper) to the patient's skin.

A dressing such as a gauze dressing can be placed over the stoma to prevent leakage from the stoma and protect both the stoma and the feeding tube. Bandage **78** can cover the dressing and provide pressure to the stoma site protection device, the underlying dressing and the feeding tube. The bandage can be a flexible adhesive bandage and fabricated from silicone, for example. It can be taken off to change the gauze and access the feeding tube. The bandage can have a larger surface area than the bumper and can have an annular disc shape.

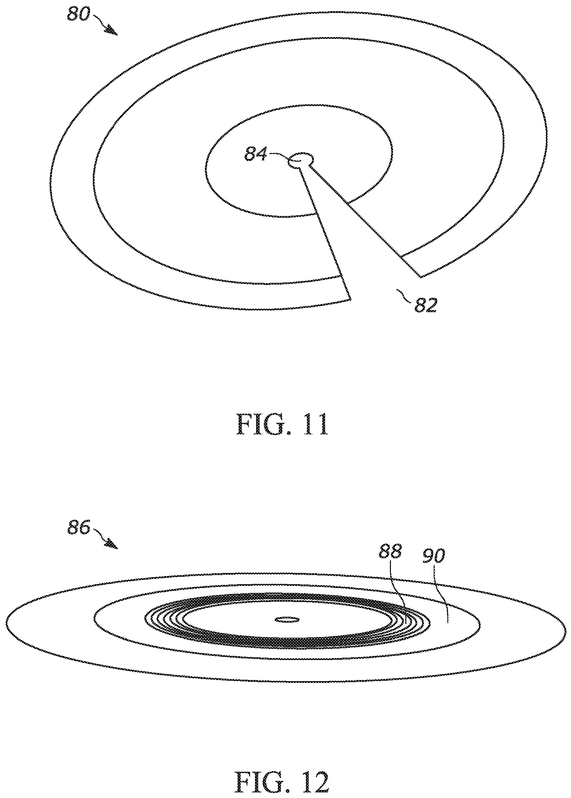

Referring to FIG. 11, in certain aspects, a bandage **80** can have a lateral slit **82** extending from and in fluid communication with a medial aperture **84**. A slit can allow a patient or caregiver to place the bandage around the tube just below the proximal end or head of the tube. This allows for the smallest requisite aperture in the bandage and maximal coverage of the bandage over the bumper to assist with securing the device in place. By pulling apart the ends of the bandage defining the slit, a user can remove the bandage from the stoma site without having to remove the feeding tube. When replacing the bandage, the user can overlap the ends of the bandage defining the slit to securely adhere the bandage to the stoma site.

Referring to FIG. 12, bandage **86** can have a plurality of ribs **88** on top surface **90** thereof to provide sufficient points of contact for a final layer of an adhesive tape or covering to be positioned on the bandage. In particular, ribs **88** can provide enough surface area for proper adhesion of a final covering layer and can reduce tugging during removal of the final covering layer.

The bandage or other component that is placed over the stoma site protection device can include a moisture-sensitive, color-changing ink incorporated into the bandage or other protective component (such as an external bumper, for example) that changes color in the presence of liquid moisture. Incorporation of such an ink can alert a user or caretaker that fluid is leaking from the stoma. In the event that fluid, such as bile, does exit the stoma, the bandage can absorb such fluid before such fluid damages the stoma site. The bandage or other component that is placed over the stoma site protection device (such as an external bumper) can have a pH sensor to detect the presence of gastric acid, which could also indicate leakage from the stoma.

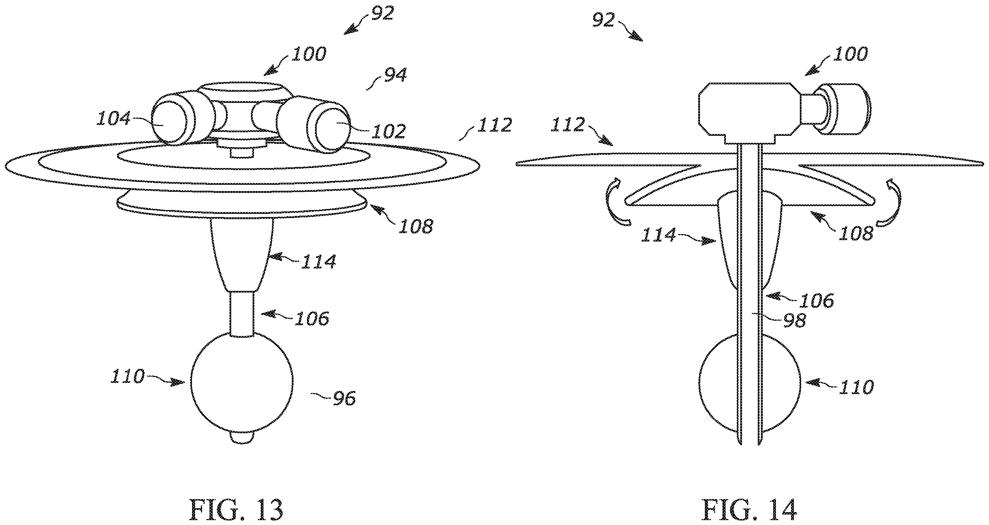

The application also discloses a feeding tube assembly is provided with a stoma site protection device and other optional components that can improve the functionality of the feeding tube. Referring to FIGs. 13-14, a feeding tube assembly **92** can comprise a proximal portion **94,** a distal portion **96,** and a lumen **98** extending longitudinally therebetween. Proximal portion **94** can comprise a proximal adapter **100** comprising one or more ports **102** and **104.** Port **102** can be a patient or caregiver access port and can be longer than port **104** making it easier for the patient or caregiver to handle. Port **104** can be shorter than port **102** and designated for use by a doctor. A feeding tube **106** can extend distally from proximal adapter **100.** A first bumper **108** can be disposed about tube **106** below proximal adapter **100** and a second bumper **110** can be disposed about tube **106** below first bumper **108** at distal portion **96**. First bumper **108** can be dome shaped and flexible to facilitate compression of the bumper when a bandage **112** is applied over first bumper **108** (the arrows in FIG. 14 schematically illustrate the flexibility of first bumper **108**. Second bumper **110** can be an expandable balloon or other structure that can positioned against the opening in the stomach or other internal structure. In use, the first bumper sits against the proximal end of the stoma tract on the outer surface of the user's skin and the second bumper sits on the distal end of the stoma tract on the inside surface of the stomach or intestines. Feeding tube assembly **92** can include a stoma site protection device comprising a non-rigid or semi-rigid plug **114** disposed about tube **106** between first bumper **108** and second bumper **110**. The stoma site protection device can have a tapered configuration and can be inflated to plug and block the stoma opening. The feeding tube assembly can also include a flexible adhesive bandage **112** that is configured to be placed against the top surface of the first bumper. Bandage **112** can have a tapered design as shown in FIG. 14 to prevent or minimize snagging or interference with the patient's clothing, for example.

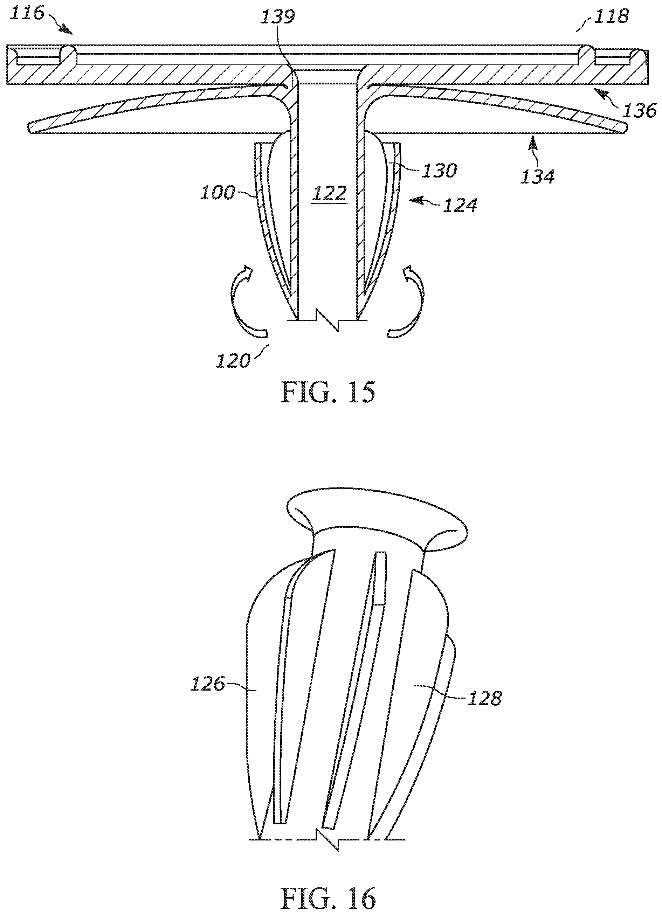

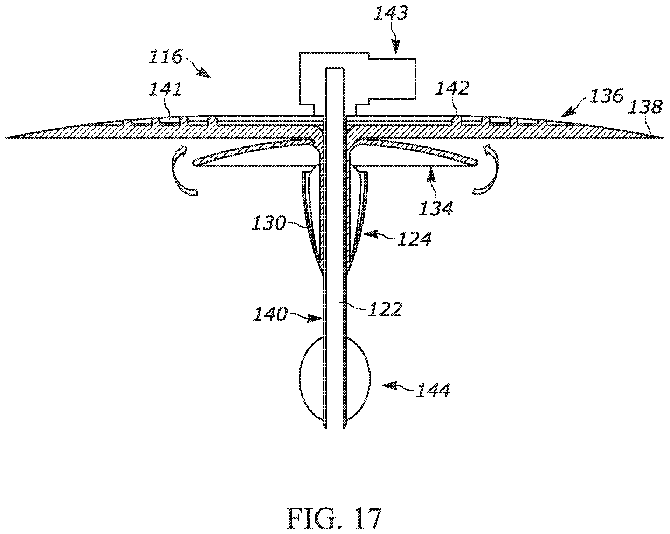

The application also discloses a stoma site protection device is part of an integrated single one-piece device that can plug the stoma tract and stabilize and secure a feeding tube assembly or other medical tube assembly. Referring to FIGs. 15-17, a device **116** can have a proximal portion **118,** a distal portion **120** and a lumen **122** extending longitudinally therethrough. Distal portion **120** can comprise a stoma site protection device **124** that has an inner portion **126** with supporting flexible ribs **128** and is surrounded by a tapered cup **130** that has a flexible outer surface. During insertion into the stoma tract, the ribs can compress and then self-expand within the stoma tract as the taper cup inverts over the ribs as indicated by the arrows in FIG. 15. Device **116** can further comprise a proximal bumper **134** that is above and integral with stoma site protection device **124** and a bandage **136** that is above and integral with proximal bumper **134**. Bumper **134** can be dome-shaped and flexible so that it is compressed as bandage **136** is applied against the user's skin (the arrows in FIG. 17 schematically illustrate the flexibility of proximal bumper **134.** Bandage **136** can have a tapered surface **138** as shown in FIGs. 17. Bandage **136** can have a tapered port **139** aligned with longitudinally extending lumen **122** of device **116** to aid in feeding tube ingress. FIG. 17 illustrates a feeding tube **140** inserted within longitudinally extending lumen **122** of device **116.** The top surface **142** of bandage **136** can have a plurality of ribs **141** to provide sufficient points of contact for a final layer of an adhesive tape or covering to be positioned on the bandage. The points of contact also reduce the effort to remove the adhesive covering from the bandage and the user's skin. Device **116** can be part of a feeding tube assembly that includes feeding tube **140,** a proximal adapter **143,** and a distal bumper **144** as illustrated in FIG. 17.

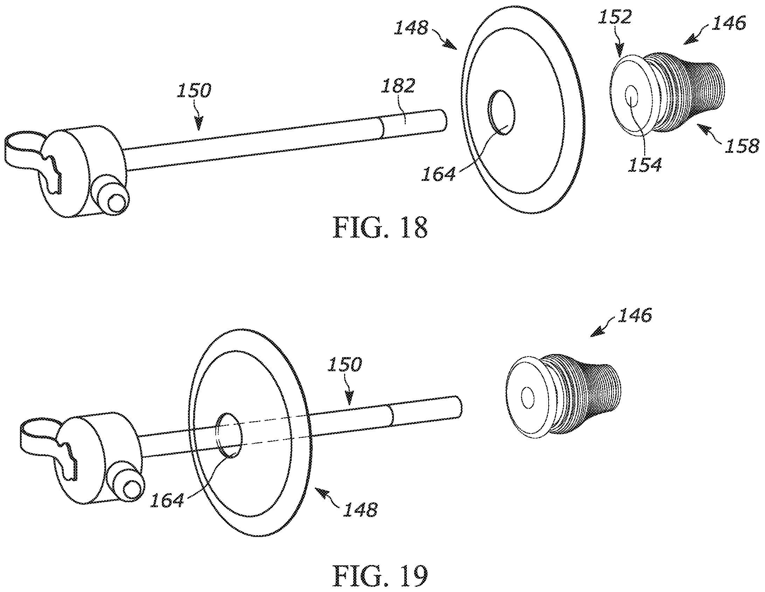

FIG. 18-21 depict images of a stoma site protection device and accompanying components during different steps of assembly with respect to a feeding tube, for example. FIG. 18 depicts a stoma site protection device **146** aligned with a bumper **148** and a feeding tube **150** having an internal retention element, such as a balloon **182** (shown inflated in FIGs. 24-27). Stoma site protection device can comprise a body having a proximal end, a distal end, an outer surface, an inner bearing surface, and a lumen extending longitudinally therethrough. The body can comprise a proximal collar **152** having a collar width and defining a top opening **154.** An indented portion can extend distally from proximal collar **156** and can have an indented portion diameter that is less than the collar diameter. A plug **158** can extend distally from the indented portion and can have an inner bearing surface that defines a bottom opening **160** and a plug lumen **162.** Plug lumen **162** can be axially aligned and in fluid communication with bottom opening **160** and top opening **154**. Bumper **148** can be dome-shaped, for example, and can define a substantially central opening **164**. Feeding tube **150** can be inserted into opening **164** of bumper **148,** top opening **154** of collar **152,** plug lumen **162,** and through bottom opening **160** as shown in FIGs. 19 and 20. Because collar **152** is flexible, it can be urged through opening **164** of bumper **148** such that bumper **148** is positioned about indented portion **149** of stoma site protection device as shown in FIG. 21, which illustrates a fully assembled stoma site protection device **146,** bumper, **148,** and feeding tube **150.**

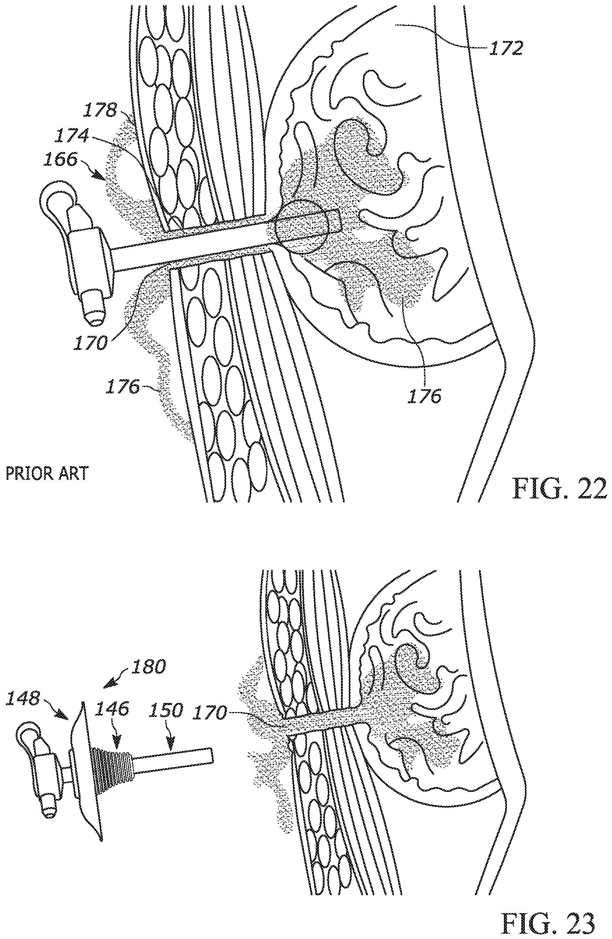

Methods of using a stoma site protection device are also provided herein. FIG. 22 illustrates a prior art feeding tube **166** inserted into a stoma **170** with the proximal end of the feeding tube at the stoma site and the distal end of feeding tube **166** in the stomach **172.** As stated above, without wishing to be bound by theory, it is believed a significant cause of leakage around a stoma site is the friction from the tube rubbing against the skin at the stoma site during regular activity causing irritation and the break-down of skin eventually creating a fissure **174** (slightly exaggerated in FIG. 22 of the purposes of illustration) through which bile or gastric acid **176** can leak out of stoma **170** to the patient's skin outer surface **178.** FIGs. 23-26 illustrate a method of using a stoma site protection device with a feeding tube to prevent or mitigate the leakage of fluid from stoma **170.** Referring to FIG. 23, an assembled stoma site protection device **146,** bumper **148,** and feeding tube **150** (referred to herein with respect to FIGs. 23-27 as an "assembly **180"** and as depicted in FIG. 21) is aligned with stoma **170.** Referring to FIG. 24, stoma site protection device **146** and feeding tube **150** are inserted through stoma **170.** Plug **158** is positioned in the subcutaneous fat layer **188** above the patient's muscle layer **190** (and/or fascia layer **151**) and below skin outer surface **178,** balloon **182** of feeding tube **150** is positioned in stomach **172,** and bumper **148** is positioned over stoma **170.** Bumper **148** sits externally on the patient's outer skin surface **178** and is compressed over stoma **170** putting the entire assembly under tension to prevent undesirable movement of assembly **180.** The internal retention element of feeding tube **150** (illustrated in the presently described figures as a balloon **182**) is inflated using a syringe **184,** for example, to secure the distal end of feeding tube in stomach **172.** As schematically illustrated in FIG. 25, because stoma site protection device **146** effectively acts as a bearing, there is no friction between the patient's skin outer surface **178** and feeding tube **150.** As such, there is no break-down of skin **178** such that a fissure is created between the stomach and the stoma. With no such fissure, there is no leakage of bile or gastric acid **176** as is the case with prior art feeding tube **166** illustrated in FIG. 22. Referring to FIGs. 25-27, a bandage **186** is placed over collar **152** of stoma site protection device **146** and bumper **148** to secure stoma site protection device **146** and bumper **148** to the patient's skin.

As stated above, devices, kits, assemblies and methods as described herein can be used for medical tubes, such as percutaneous tubes, that are inserted into a stoma. Such tubes include feeding tubes that are placed through the nose, including nasogastric, nasoduodenal, and nasojejunal tubes; or placed directly into the abdomen, such as a gastrostomy, gastrojejunostomy, or jejunostomy feeding tube. Other medical tubes include chest tubes and surgical drains. Chest tubes include tubes used with ventricular assist devices including left and/or right ventricular assist devices. Non-limiting examples of dimensions of low profile feeding tubes is a length of between about 1.0 centimeters (cm) to about 4.5 cm and a tube diameter of between about 12 French (Fr) to about 24 Fr. Non-limiting examples of dimensions for adult chest tubes are about 20 Fr to about 40 Fr and about 6 Fr to about 26 Fr for children chest tubes. Non-limiting examples of dimensions for percutaneous drainage tubes are about 6.5 Fr to about 20 Fr.

Stoma site protection devices as disclosed herein prevent acid or other fluids from leaking out of the tube, provide pressure on the tube, and can be released from the tube in order to remove pressure or to allow drainage. The plug can have a depth such that it prevents acid or other fluid from accumulating near the upper end of the skin. As such, a stoma site protection device can prevent skin irritation surrounding the stoma. In addition, the plug does not have a depth so great that it compromises the seal between the feeding tube and the internal site, such as the stomach or intestine.

## Claims

1. A stoma site protection device (10) comprising:
a body (11) having a proximal end (12), a distal end (14), an outer surface (15), an inner bearing surface (17), and a lumen (19) extending longitudinally therethrough, the body comprising:
a proximal collar (16) having a collar width (W₁) and defining a top opening (18);
an indented portion (20) extending distally from the proximal collar (16) and having an indented portion width (W₂) that is less than the collar width (W₁); and
a silicone plug (22) extending distally from the indented portion and having
an outer surface (24) and an inner bearing surface (27),
a bottom opening (26) and
a plug lumen (29) axially aligned and in fluid communication with the bottom opening (26) and the top opening (18), the stoma site protection device (10) being sterile and being sized and dimensioned to prevent or minimize leakage of fluid from a stoma;
**characterized in that** the inner bearing surface (27) of the silicone plug (22) has a Shore durometer hardness greater than the outer surface (24) of the silicone plug (22).

2. The stoma site protection device (10) of claim 1, wherein the outer surface (24) of the silicone plug (22) includes ridges (30) about a circumference thereof.

3. The stoma site protection device (10) of claim 1, wherein the silicone plug (22,34) has an upper portion (36) and a lower portion (38), the upper portion (36) having a bulbous shape, the lower portion (38) tapering from a top end (40) to a bottom end (42) of the lower portion (38).

4. The stoma site protection device (10) of claim 1, wherein the indented portion (20, 45) and the silicone plug (22, 47) have substantially the same uniform diameter along the length of the indented portion (20, 45) and the silicone plug (22, 47).

5. A stoma site protection kit comprising:
the stoma site protection device (10) of any preceding claim and further comprising a bumper (72) defining an opening (74) extending longitudinally therethrough, the opening (74) sized and configured to be placed about the indented portion of the body of the stoma site protection device (10).

6. The stoma site protection kit of claim 5, wherein the bumper (72) is flexible and has a dome-shaped portion (75) surrounding the opening, the dome-shaped portion surrounded by a flange (76).

7. The stoma site protection kit of claim 5, further comprising an adhesive bandage (78) defining an opening (80) extending longitudinally therethrough and sized and configured to be placed about the bumper (72).

8. The stoma site protection kit of claim 7, wherein the opening (80) of the adhesive bandage (78) is in fluid communication with a laterally extending slit (84).

9. The stoma site protection kit of claim 7, wherein the adhesive bandage (78,86) has a top surface (90) comprising a plurality of ribs (88).

10. The stoma site protection kit of claim 7, wherein the adhesive bandage (78,86) has a surface area greater than the surface area of the bumper (72).

11. The stoma site protection kit of claim 7, wherein the adhesive bandage (78,86) has a moisture-sensitive, color-changing ink incorporated therein.

## Patentansprüche

1. Stomastellen-Schutzvorrichtung (10), Folgendes umfassend:
einen Körper (11) der ein proximales Ende (12), ein distales Ende (14), eine Außenfläche (15), eine innere Auflagefläche (17) und ein Lumen (19) aufweist, das sich in Längsrichtung durch diesen hindurch erstreckt, wobei der Körper Folgendes umfasst:
einen proximalen Kragen (16), der eine Kragenbreite (W₁) aufweist und eine obere Öffnung (18) definiert;
einen eingekerbten Abschnitt (20), der sich distal von den proximalen Kragen (16) erstreckt und eine Breite (W₂) aufweist, die geringer ist als die Breite (W1) des Kragens; und
einen Silikonstopfen (22), der sich distal von dem eingekerbten Abschnitt erstreckt und
eine Außenfläche (24) und eine innere Lagerfläche (27),
eine Bodenöffnung (26) und
ein Stopfenlumen (29) aufweist, das axial ausgerichtet ist und in Fluidverbindung mit der unteren Öffnung (26) und der oberen Öffnung (18) steht, wobei die Stomastellen-Schutzvorrichtung (10) steril ist und so bemessen ist, dass sie das Austreten von Fluid aus einem Stoma verhindert oder minimiert;
**dadurch gekennzeichnet, dass** die innere Auflagefläche (27) des Silikonstopfens (22) eine größere Shore-Durometer-Härte aufweist als die Außenfläche (24) des Silikonstopfens (22).

2. Stomastellen-Schutzvorrichtung (10) nach Anspruch 1, wobei die Außenfläche (24) des Silikonstopfens (22) an ihrem Umfang Rippen (30) beinhaltet.

3. Stomastellen-Schutzvorrichtung (10) nach Anspruch 1, wobei der Silikonstopfen (22, 34) einen oberen Abschnitt (36) und einen unteren Abschnitt (38) aufweist, wobei der obere Abschnitt (36) eine bauchige Form aufweist und der untere Abschnitt (38) sich von einem oberen Ende (40) zu einem unteren Ende (42) des unteren Abschnitts (38) verjüngt.

4. Stomastellen-Schutzvorrichtung (10) nach Anspruch 1, wobei der eingekerbte Teil (20, 45) und der Silikonstopfen (22, 47) im Wesentlichen den gleichen gleichmäßigen Durchmesser entlang der Länge des eingekerbten Teils (20, 45) und des Silikonstopfens (22, 47) aufweisen.

5. Stomastellen-Schutzset, Folgendes umfassend:
die Stomastellen-Schutzvorrichtung (10) nach einem der vorhergehenden Ansprüche und ferner umfassend einen Stoßfänger (72), der eine Öffnung (74) definiert, die sich in Längsrichtung durch ihn hindurch erstreckt, wobei die Öffnung (74) so bemessen und konfiguriert ist, dass sie um den eingekerbten Abschnitt des Körpers der Stomastellen-Schutzvorrichtung (10) herum platziert werden kann.

6. Stomastellen-Schutzset nach Anspruch 5, wobei der Stoßfänger (72) flexibel ist und einen kuppelförmigen Abschnitt (75) aufweist, der die Öffnung umgibt, wobei der kuppelförmige Abschnitt von einem Flansch (76) umgeben ist.

7. Stomastellen-Schutzset nach Anspruch 5, ferner umfassend einen Klebeverband (78), der eine Öffnung (80) definiert, die sich in Längsrichtung durch ihn hindurch erstreckt, und der so bemessen und konfiguriert ist, dass er um den Stoßfänger (72) platziert werden kann.

8. Stomastellen-Schutzset nach Anspruch 7, wobei die Öffnung (80) des Klebeverbands (78) in Fluidverbindung mit einem seitlich verlaufenden Schlitz (84) steht.

9. Stomastellen-Schutzset nach Anspruch 7, wobei der Klebeverband (78, 86) eine Oberseite (90) aufweist, die eine Vielzahl von Rippen (88) umfasst.

10. Stomastellen-Schutzset nach Anspruch 7, wobei der Klebeverband (78, 86) eine größere Oberfläche als die Oberfläche des Stoßfängers (72) aufweist.

11. Stomastellen-Schutzset nach Anspruch 7, wobei in den Klebeverband (78, 86) eine feuchtigkeitsempfindliche, farbwechselnde Tinte eingearbeitet ist.

## Revendications

1. Dispositif de protection (10) de site de stomie, comprenant :
un corps (11) ayant une extrémité proximale (12), une extrémité distale (14), une surface externe (15), une surface d'appui interne (17), et une lumière (19) s'étendant longitudinalement à travers ce dernier, le corps comprenant :
un collet proximal (16) ayant une largeur (W₁) de collet et définissant une ouverture supérieure (18) ;
une partie en retrait (20) s'étendant distalement à partir du collet proximal (16) et ayant une largeur (W₂) de partie en retrait qui est inférieure à la largeur (W₁) du collet ; et
un bouchon silicone (22) s'étendant distalement à partir de la partie en retrait et ayant
une surface externe (24) et une surface d'appui interne (27),
une ouverture inférieure (26) et
une lumière (29) de bouchon alignée axialement et en communication fluidique avec l'ouverture inférieure (26) et l'ouverture supérieure (18), le dispositif de protection (10) de site de stomie étant stérile et étant conçu de taille et dimensions pour prévenir ou réduire au minimum la fuite de fluide à partir d'une stomie ;
**caractérisé en ce que** la surface d'appui interne (27) du bouchon silicone (22) présente une plus grande dureté au duromètre Shore que la surface externe (24) du bouchon silicone (22).

2. Dispositif de protection (10) de site de stomie selon la revendication 1, dans lequel la surface externe (24) du bouchon silicone (22) inclut des crêtes (30) autour d'une circonférence de ce dernier.

3. Dispositif de protection (10) de site de stomie selon la revendication 1, dans lequel le bouchon silicone (22, 34) présente une partie supérieure (36) et une partie inférieure (38), la partie supérieure (36) ayant une forme bulbeuse, la partie inférieure (38) s'amincissant à partir d'une extrémité supérieure (40) vers une extrémité inférieure (42) de la partie inférieure (38).

4. Dispositif de protection (10) de site de stomie selon la revendication 1, dans lequel la partie en retrait (20, 45) et le bouchon silicone (22, 47) ont sensiblement le même diamètre uniforme le long de la longueur de la partie en retrait (20, 45) et du bouchon silicone (22, 47).

5. Kit de protection de site de stomie, comprenant :
le dispositif de protection (10) de site de stomie selon l'une quelconque des revendications précédentes et comprenant en outre un amortisseur (72) définissant une ouverture (74) s'étendant longitudinalement à travers ce dernier, l'ouverture (74) étant dimensionnée et configurée pour être placée autour de la partie en retrait du corps du dispositif de protection (10) de site de stomie.

6. Kit de protection de site de stomie selon la revendication 5, dans lequel l'amortisseur (72) est flexible et présente une partie (75) en forme de dôme entourant l'ouverture, la partie en forme de dôme étant entourée d'une collerette (76).

7. Kit de protection de site de stomie selon la revendication 5, comprenant en outre un bandage adhésif (78) définissant une ouverture (80) s'étendant longitudinalement à travers ce dernier et dimensionné et configuré pour être placé autour de l'amortisseur (72).

8. Kit de protection de site de stomie selon la revendication 7, dans lequel l'ouverture (80) du bandage adhésif (78) est en communication fluidique avec une fente (84) s'étendant latéralement.

9. Kit de protection de site de stomie selon la revendication 7, dans lequel le bandage adhésif (78, 86) a une surface supérieure (90) comprenant une pluralité de nervures (88).

10. Kit de protection de site de stomie selon la revendication 7, dans lequel le bandage adhésif (78, 86) a une plus grande superficie que la superficie de l'amortisseur (72).

11. Kit de protection de site de stomie selon la revendication 7, dans lequel le bandage adhésif (78, 86) comporte une encre changeant de couleur, sensible à l'humidité, incorporée dans ce dernier.
